# EUROPEAN PATENT APPLICATION

(11) **EP 0 836 865 A1**
(43) Date of publication of application: **22.04.1998**
(21) Application number: 96905091.3
(22) Date of filing: 21.02.1996
(51) Int. Cl.: A61N 1/14, A61H 39/00, A43B 7/36, A41B 11/00

(54) **METHOD OF REFLEXOTHERAPY AND A REFLEXOTHERAPY DEVICE (VARIANTS)**

(30) Priority: 19.05.1995 RU 95108741; 19.05.1995 RU 95108742; 19.05.1995 RU 95108744
(71) Applicant: Paskar, Igor Petrovich, Moscow, 121002 (RU)
(72) Inventor: Paskar, Igor Petrovich, Moscow, 121002 (RU)
(74) Representative: Beetz & Partner Patentanwälte
(86) International application number: RU9600040
(87) International publication number: WO9636392

(57) **Abstract**

The invention pertains to medicine, in particular reflexotherapy. Its purpose is to normalise the electromagnetic and electrostatic parameters of the human organism for prophulactic and therapeutic purposes. The method involves earthing particular or all reflex zones of the surfases of the soles of the feet. The proposed reflexotherapy device can take the form of foot platforms provided with electrical contacts and mounted on a base, shoes, insoles or stocking articles in which the part in contact with the surface of the sole is provided with electrically conducting areas which facilitate earthing of the particular reflex zones distributed over the surface of the sole or occupying the entire sole surface.

## Description

### Field of the Invention

The invention relates to medicine, in particular, to reflexotherapy.

### Background Art

Reflexotherapy is based on external action on reflex points or skin zones of a human being, hereinafter referred to as RZ. Different methods and devices for such action exist, e.g. acupuncture, current, magnetic field, laser radiation, mechanical action, heat, etc.

A method for health improvement by grounding the whole surface of the soles of feet is known. This method is one of the parts of the P.K. Ivanov natural health improvement system "Detka." "Before you bathe, or after, or (if possible) while you are still bathing, go outdoors barefooted and stand on the ground or in winter on snow, for at least 1-2 minutes" (V.S. Orlin, "The Teaching of Porfirii Ivanov 'Detka'," Moscow, Sovexportkniga, 1991).

The method suffers from some drawbacks, among which:
- It is impossible or inadvisable for the overwhelming majority of urban residents to use this part of the natural health improvement system as proposed by P.K. Ivanov;
- The method does not make it possible to ground separate RZ located on the surface of soles of feet.

The following methods and devices are those most similar to the invention.

A device for acting on the reflex points of the sole (realizing the method for reflexotherapy). The device is provided with foot platforms. Electroconductive zones are provided on the platforms and positioned so that these zones contact with the RZ when feet are put on the platforms. The electroconductive sections of a platform are electrically connected to a commutator, making it possible to act on the RZ with electric current (Application DE 3103502, class A61H 39/00, 1982).

A device providing massage and electrostimulation of reflex points on the sole and consisting of movable upper and lower discs, a laid-on flexible disc with recesses for the feet in which there are massage lugs and pressed therein contact metal balls, fixation lugs, a gasket to commutate negative charges, a perforated insulating gasket, piezoelectric ferroelectric elements, and connecting conductors (Inventor's Certificate SU 1599013, class A61H 39/00, 1990).

These devices do not make it possible to ground separate reflex zones because there is no grounding conductor.

The use of various types of electroconductive footwear in electrical power engineering, electronics, chemistry, rubber engineering and other branches of industry where the static electrization of materials and service personnel and the necessity of preventing accumulation of electrostatic electrization on the bodies of people arise.

The following may be regarded as analogues:
- Electroconductive footwear - Inventor's Certificate 244910, class A43B, 1969;
- Electroconductive footwear - Inventor's Certificate SU 1732921, class A43B, 1989.

This type of footwear does not make it possible to ground separate reflex zones which are located on the soles of feet and are necessary in the case of concrete pathologies.

Different types of therapeutic insoles are known whose purpose is to act on reflex points (zones) located on the surfaces of foot soles.

These are:
- Insoles provided with massage elements, the construction of which makes it possible to rearrange the massage elements to act on one or another RZ on the soles (Inventor's Certificate SU 1713580, class A43B 7/36, 1991):
- Therapeutic insoles, the construction of which includes an electronic unit, active electrodes and insoles with holes, which makes it possible to rearrange the active electrodes into contact with corresponding RZ on the soles (Inventor's Certificate SU 1819608, class A61H 39/00. 1993).

The construction of the insoles described above does not make it possible to ground separate reflex zones because there are no grounding conductors.

Various types of hosiery are known which are a formed sheathing made of natural or synthetic fibre. The sheathing repeats the form and size of a human foot. Up to the present time no requirements were made regarding the electrical conductivity of hosiery. Existing hosiery can gain an electroconductive property as a result of the moisture released when the foot sweats. However, due to large electrical resistance and the absence of facilities making it possible to ground one or several RZ of the soles of feet while simultaneously insulating other RZ of the soles of feet from ground, they cannot perform grounding of separate or all the RZ of the soles of feet.

### Disclosure of the Invention

The proposed invention is aimed at solving the problem of normalization of electromagnetic and electrostatic body characteristics by means of reflexotherapy which consists of grounding concrete reflex zones located on the surfaces of soles of a human being's feet and corresponding to an organ (organs) having a pathology, and also for prophylaxis by grounding the whole surface of the feet soles.

This problem is solved in a method for reflexotherapy consisting of acting on RZ located on the surfaces of the soles of feet, the action being performed by grounding at least one RZ corresponding to a concrete pathology.

In certain cases of carrying out the method, several or all of the RZ located on the surface of feet soles are grounded, or the whole surface of the soles of the feet are grounded.

The problem is also solved in a means for reflexotherapy comprising a base with foot platforms disposed thereon, contacts being located on the platforms, which contacts are electrically connected to a commutation unit, which in turn is provided with a grounding conductor and is made adaptable for electrically connecting that conductor to a contact or group of contacts, the location of which coincides with the location of at least one RZ located on the surface of the soles of feet and corresponding to a certain pathology.

Wherein the commutation unit may be made adaptable for electrical connection of the grounding conductor with all the contacts simultaneously.

In order to ensure a guaranteed contact of any RZ on the soles of feet, independent of the size of the foot, the size of the electrical contacts and spacing between them are selected in such a manner that any RZ located on a foot surface corresponds to at least one electrical contact.

The problem is also solved in that the means for reflexotherapy is made in the form of footwear consisting of an upper part and connected thereto sole, wherein the sole is made of electroinsulating material and has electroconductive zones on its surface, the size and location of which coincide with the size and location of at least one RZ located on the surface of the soles of feet and corresponding to a certain pathology.

In another variant the means for reflexotherapy is made in the form of footwear consisting of an upper part, connected therewith electroconductive sole and a supplementary insole, wherein the insole is made of an electroinsulating material and has electroconductive zones on its surface, the size and location of which zones coincide with the size and location of at least one RZ located on the surface of the soles of feet and corresponding to a certain pathology.

In another variant the means is made in the form of footwear consisting of an upper part, connected therewith sole and a main insole, wherein it is additionally provided with a supplementary insole made of electroinsulating material and having electrically conductive zones on its surface. The size and location of the zones coincide with the size and location of at least one RZ located on the surface of the soles of feet and corresponding to a certain pathology. The main insole is made electrically conductive over the whole surface thereof and is placed between the sole and the supplementary insole so as to ensure an electrical Contact between the electroconductive zones of the supplementary insole and the external surface of the footwear sole.

In order to ensure the prophylaxis and treatment of different illnesses, the footwear can be equipped with a set of replaceable insoles made of electroinsulating material and provided with electroconductive zones on its surface. Wherein the size and location of the electroconductive zones of each pair of replaceable insoles coincides with the size and location of at least one RZ located on the surface of the soles of feet and corresponding to a certain pathology.

The problem is also solved in that the means for reflexotherapy is made in the form of insoles for footwear which are made from electroinsulating material having electroconductive zones on its surface, the size and location of which zones coincide with the size and location of at least one RZ located on the surface of the soles of feet and corresponding to a certain pathology, wherein the electroconductive zones are made adaptable for electrical contact with the electroconductive soles of the footwear.

The electroconductive zones in all variants can be made of metal.

The problem is also solved in that the means for reflexotherapy is made in the form of hosiery, wherein the hosiery is made of electroinsulating fibre, while there are electroconductive zones on the sole part of the hosiery. The size and location of the zones coincide with the size and location of at least one RZ located on the surface of the soles of feet and corresponding to a certain pathology.

In another variant of the hosiery, the whole sole part is made electroconductive, while the remaining part of the hosiery is made of electroinsulating fibre.

In another variant of the hosiery, the whole article is made of electroconductive fibre.

The electroconductive parts of the hosiery in all variants can be made by impregnating those parts with an electroconductive adhesive composition or using electroconductive fibre.

### Brief Description of the Drawings

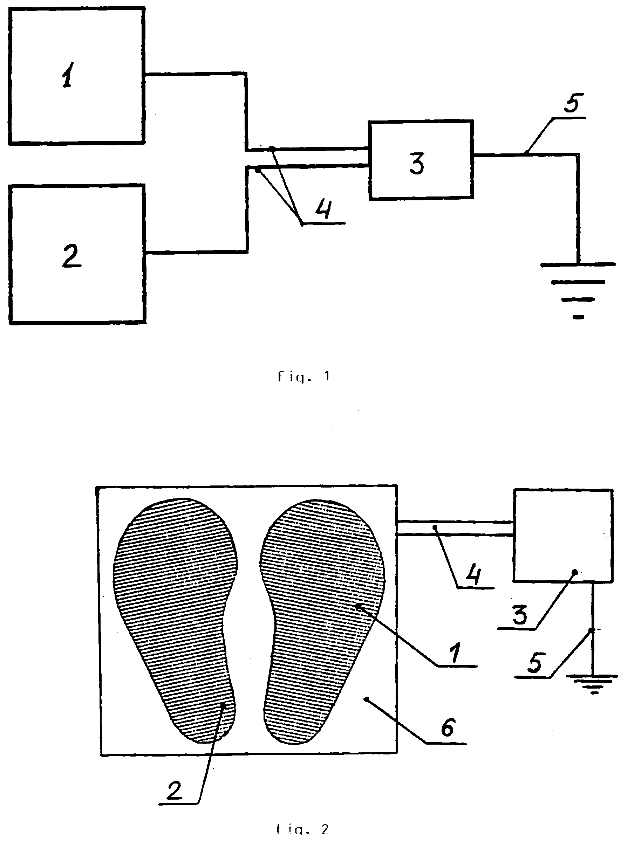

Fig. 1 shows a block diagram of a means for reflexotherapy comprising platforms for feet, which platforms are set on a base.

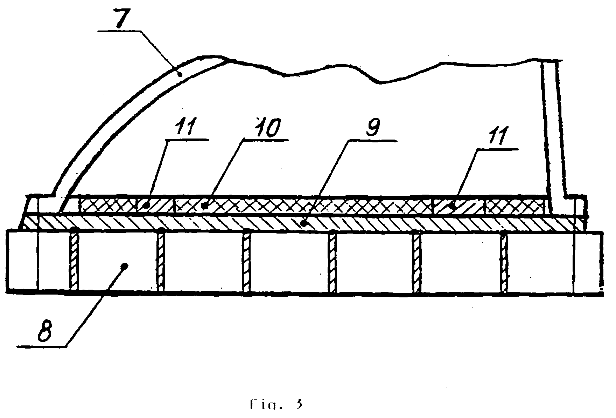

Fig. 2 shows a general view of that same device.

Fig. 3 shows a longitudinal section of a means for reflexotherapy made in the form of footwear.

### Methods of Carrying Cut the Invention

The means for reflexotherapy in one of the variants comprises contact groups 1 and 2 for both feet, a commutator unit 3, electroconductor cables 4, a grounding electroconductor 5. The contacts 1 and 2 located on foot platforms are electrically connected to the commutator unit 3 by means of the electroconductor cables 4. The commutator unit 3 is made in such a manner that it is possible to provide an electrical contact between the grounding conductor 5 and any contact or group of contacts, the location of which coincides with the size and location of at least one RZ located on the surface of soles of feet and corresponding to a certain pathology.

The means for reflexotherapy can be made in the form of footwear. The footwear consists of an upper part 7, an electroconductive sole 8, a main electroconductive insole 9 having an electrical contact with the external surface of the sole 8, and a supplementary insole 10 made of electroinsulating material and having electroconductive zones 11 which correspond in respect of size and location to the topology of the reflex zones of the soles of feet depending on the pathology which is present. When such shoes are worn without hosiery (on bare feet) or with special electroconductive hosiery, the grounding of a human organism will be effected through the RZ located on the surface of the soles of feet and in contact with the electroconductive zones on the surface of the supplementary insole 10.

### Industrial Applicability

In the process of multicentury evolution the human organism has been under the effect of ambient electromagnetic fields and grounded via foot soles. Following the logic of evolutionary development, the conclusion can be made that the RZ of the external integument of the organism play an important and differentiated role in ensuring the electromagnetic and electrostatic balance of the organism. In particular, the RZ located on the surface of the soles of feet have the purpose of grounding the organism. In the last decades the intensity of electromagnetic fields acting on the human organism has substantially increased. The wide use of synthetic materials for the manufacture of furniture, clothing, footwear, floor coverings and other household items results in accumulation of static electricity on the organism. The RZ located on the soles of feet are isolated from the ground and do not fulfill the functions of grounding which were developed for them by the evolution of development of the human organism. Grounding one, several or all of the RZ located on the surface of the soles of feet makes it possible to carry out reflexotherapeutic action on the human organism as a means for treatment or prophylaxis.

## Claims

1. A method for reflexotherapy consisting of acting on reflex zones located on the surface of soles of the feet, characterized in that the action is carried out by grounding at least one reflex zone corresponding to a certain pathology.

2. A method for reflexotherapy according to claim 1, characterized in that all the reflex zones located on the surface of the soles of the feet are grounded.

3. A method for reflexotherapy according to claim 1, characterized in that the whole surface of the soles of the feet is grounded.

4. A means for reflexotherapy comprising a base with disposed thereon platforms for the feet, on which platforms are located contacts electrically connected to a commutator unit, characterized in that the commutator unit is provided with a grounding conductor and is made adaptable for electrically connecting that conductor to a contact or group of contacts, the location of which coincides with the location of at least one reflex zone located on the surface of the soles of feet and corresponding to a certain pathology.

5. A means for reflexotherapy according to claim 4, characterized in that the commutator unit is made adaptable for electrically connecting the grounding conductor simultaneously to all the contacts.

6. A means for reflexotherapy according to claim 4, characterized in that the dimensions of the electrical contacts and the spacing between them are selected in such a manner that any reflex zone located on the surface of the soles of feet corresponds to at least one contact.

7. A means for reflexotherapy according to claim 4, characterized in that the contacts are made of metal.

8. A means for reflexotherapy made in the form of footwear consisting of an upper part and connected thereto sole, characterized in that the sole is made of an electroinsulating material and has electroconductive zones on its surface, the size and location of which zones coincide with the size and location of at least one reflex zone located on the surface of the soles of feet and corresponding to a certain pathology.

9. A means for reflexotherapy made in the form of footwear consisting of an upper part and connected thereto electroconductive sole and a supplementary insole, characterized in that the insole is made from electroinsulating material and has on its surface electroconductive zones, the size and location of which coincide with the size and location of at least one reflex zone located on the surface of the soles of feet and corresponding to a certain pathalogy.

10. A means for reflexotherapy according to claim 9, characterized in that it is additionally provided with a set of replaceable insoles made of an electroinsulating material and having on its surface electroconductive zones, wherein the size and location of the electroconductive zones of each pair of replaceable insoles coincide with the size and location of at least one reflex zone located on the surface of the soles of feet and corresponding to a certain pathology.

11. A means for reflexotherapy according to claim 9, characterized in that the electroconductive zones are made of metal.

12. A means for reflexotherapy made in the form of footwear consisting of an upper part, connected thereto sole and main insole, characterized in that it is additionally provided with a supplementary insole made of electroinsulating material and having on its surface electroconductive zones, the size and location of which coincide with the size and location of at least one reflex zone located on the surface of the soles of feet and corresponding to a certain pathology, wherein the main insole is made electrically conductive over its whole surface and is disposed between the sole and the supplementary insole to ensure an electrical contact between the electroconductive zones of the supplementary insoles and the external surface of the sole.

13. A means for reflexotherapy according to claim 12, characterized in that it is additionally provided with a set of replaceable insoles made from electroinsulating material and having on the surface thereof electroconductive zones, wherein the size and location of the electroconductive zones of each pair of replaceable insoles coincide with the size and location of at least one reflex zone located on the surface of the soles of feet and corresponding to a certain pathology.

14. A means for reflexotherapy according to claim 12, characterized in that the electroconductive zones are made of metal.

15. A means for reflexotherapy made in the form of insoles of electroinsulating material and having on the surface thereof electroconductive zones, characterized in that the electroconductive zones are made adaptable for contact with an electroconductive sole of footwear, wherein the size and location of electroconductive zones coincide with the size and location of at least one reflex zone located on the surface of the soles of feet and corresponding to a certain pathology.

16. A means for reflexotherapy according to claim 15, characterized in that the electroconductive zones are made of metal.

17. A means for reflexotherapy made in the form of hosiery, characterized in that the hosiery is made of electroinsulating fibre, and electroconductive zones are made on the sole part, the size and location of which zones coincide with the size and location of at least one reflex zone located on the surface of the soles of feet and corresponding to a certain pathology.

18. A means for reflexotherapy according to claim 17, characterized in that the electroconductive zones are made by impregnating the hosiery in those zones with an electroconductive adhesive composition.

19. A means for reflexotherapy according to claim 17, characterized in that the electroconductive zones are made of electroconductive fibre.

20. A means for reflexotherapy made in the form of hosiery, characterized in that the sole part of the hosiery is made electroconductive, and the remaining part of the hosiery is made of electroinsulating material.

21. A means for reflexotherapy according to claim 20, characterized in that the sole part of the hosiery is made electroconductive by impregnating that part with an electroconductive adhesive composition.

22. A means for reflexotherapy according to claim 20, characterized in that the sole part of the hosiery is made of electroconductive fibre.

23. A means for reflexotherapy made in the form of hosiery, characterized in that the whole of the hosiery is made of electroconductive fibre.
